# EUROPEAN PATENT APPLICATION

(11) **EP 4 111 850 A1**
(43) Date of publication of application: **04.01.2023**
(21) Application number: 21182052.7
(22) Date of filing: 28.06.2021
(51) Int. Cl.: A01G 9/02, A01G 27/00, G01N 31/22, G01N 33/24

(54) **A COLOR-BASED HUMIDITY INDICATOR IN A PLANT CULTIVATION CONTAINER**

(71) Applicant: Freshape SA, 1690 Villaz-St-Pierre (CH)
(72) Inventor: LIU, Yuhang, 1030 Bussigny (CH); LIU, Yizhu, 1030 Bussigny (CH)
(74) Representative: Schneiter, Sorin

(57) **Abstract**

The present invention concerns an arrangement comprising a container and a material that has the property of changing color upon exposure to humidity or to liquid water. In an embodiment, the material is directly coated onto an inner surface of a transparent side wall part of said container. Preferably, the material is a hydrochromic material. Preferably, the arrangement is used for the cultivation of plants in the container, preferably for crop cultivation in glasshouses.

## Description

### Technical Field

The present invention generally relates to the field of cultivation and crop growing. More specifically, the invention relates to the containers for the cultivation of plants and to the use of a material with such a container, wherein the material has the property of changing color upon exposure to humidity or to liquid water. The invention further relates the use of the container and the material in crop cultivation, to a method for cultivating a plant and to a system suitable for monitoring water status in the cultivation of plants.

### Background Art and Problems Solved by the Invention

In the field of cultivation and crop growing, the management of the supply of water, and possibly nutrients dissolved in the water, to a crop plant remains a challenge. In order to optimize plant grow and/or yield, it is necessary to understand the water need of a plant and to control the water supply adequately. It is well known that both excess of water and water shortage can be deleterious to the plant's health.

The above problem applies also to crop plants grown in glass houses or more generally grown in plant pots or containers, for example plants grown in hydroculture or hydroponics.

In view of the above, it is an objective of the present invention to provide a means for obtaining information about the water status of a plant, in particular in view of timing the watering of the plant in an optimized manner.

It is also an objective of the invention to obtain said information easily, rapidly and reliably. It is in particular an objective to obtain the water status in plant container in a manner that can be easily understood by a human farmer, but which information is also be susceptible of being accessible to automation. It is an objective of the invention to provide information on the water status in a plant container, wherein said information can be read in an automated manner, also in the absence of a human operator. Ideally, the information can be used by an automated watering system, which can thus adequately water the plant in an automated manner, depending on the water status in a plant container.

The present invention addresses the objectives set out above.

### Summary of the Invention

The present inventors plant cultivation using materials that have the property of changing their color in dependence of water or moisture exposure. The materials are useful for monitoring the water status of containers in which said plant is grown.

In an aspect, the invention provides a container for the cultivation of a plant, the container comprising at least one transparent wall part and a material that has the property of exhibiting a particular color depending on the humidity to which said material is exposed, wherein said material is associated with said container so as to be visible through said transparent wall part.

In an aspect, the invention provides the use of an arrangement comprising a substrate and a material having the property of exhibiting a particular color depending on the humidity to which said material is exposed for indicating water status in the cultivation of a plant. Preferably, said cultivation is soilless culture and/or hydroculture, for example hydroponics.

In an aspect, the invention provides a method for cultivating a plant, the method comprising: providing the container according to the invention; cultivating a plant in said container; monitoring humidity and/or water status in said container by monitoring the color and/or wavelength of the light reflected by said material.

In an aspect, the invention provides a system suitable for monitoring water status in the cultivation of plants, the system comprising: the container of the invention; a sensor provided to capture light reflected by the hydrochromic coating associated with said container; a data processing entity configured to determine, from a signal produced by said light sensor, the humidity and/or water status in said container and/or water requirements of a plant cultivated in said container.

Further aspects and preferred embodiments of the invention are defined herein below and in the appended claims. Further features and advantages of the invention will become apparent to the skilled person from the description of the preferred embodiments given below.

### Brief Description of the Drawings

**Figure 1** shows the chemical structures of the starting materials used to prepare the material in accordance with the first embodiment.
**Figure 2** shows the chemical structures of the starting materials used to prepare the material in accordance with the second embodiment.
**Figure 3** shows the color change from entirely green (left image) to red (right image) upon exposure to water of an area comprising the material of the second embodiment of the invention.
**Figure 4** shows a humidity chamber prepared to characterize the materials prepared in accordance with an embodiment of the invention.
**Figures 5** and **6** show the change of the reflectance peak position as a function of the humidity levels for the film shown in Figure **3**.
**Figure 7** shows the transmittance spectra of the films shown in **Figure 3** recorded after rinsing and drying following removal of the films from a nutrient solution at intervals.
**Figure 8** shows the trend of the characteristic reflection peak wavelength of the films shown in **Figure 7**.

Hereinafter, preferred embodiments of the device of the invention are described, in order to illustrate the invention, without any intention to limit the scope of the present invention.

### Detailed Description of the Preferred Embodiments

The present invention relates to materials that have the property of changing their color in dependence of exposure to water or moisture. In accordance with the invention, such materials are used in plant growing and more precisely, in association with a container, such as pots, recipients, planting beds, for example raised planting beds, and the like. The container may in principle have or comprise any shape, such as rectangular, conical, cylindrical, and may of course have a shape that is composed of different geometrical shapes, as is frequently the case with pots for plants, for example.

For the purpose of the present invention, the term "comprising" is intended to mean "includes, amongst other". It is not intended to mean "consists only of".

For the purpose of the present invention, the expression "material ... in association with a container" is intended to mean that the material may be connected to, for example deposited, on the container, but the expression also includes a loose association, in which the material is provided on a substrate that is not rigidly or undetachably connected to the container. The material may, for example, be provided as a piece that is separate from the container, but which is positioned more stably in the container once the container is used for cultivation. The material may, for example, be held in the container by a matrix material, such as soil or clay pebbles, which is added to the container. Accordingly, the invention provides an arrangement comprising the container and the material, wherein the material is preferably provided on a substrate.

In an embodiment, the material is provided in the form of a coating on the inner side of a transparent wall or wall part of said container. For example, the material is deposited directly on the transparent inner wall of the container, or is deposited on a transparent intermediate layer deposited on said inner wall or wall part.

In an embodiment, the material is provided on a substrate provided inside the container said substrate being visible through said transparent wall part. Said substrate may be flat, but may also have another form, such as on an object having the form of a rod or another geometric form, it being preferred that the object comprising the material is visible through the transparent wall or wall part of the container, preferably when a plant is cultivated in the container. Preferably, the material is deposited as a layer or film on said substrate.

In an embodiment, said transparent wall part is preferably a transparent side wall part, preferably a transparent side wall. The container may be only partially transparent or may be totally transparent. In an embodiment, the container is substantially made from a transparent material. In another embodiment, some walls of the container are not transparent, while at least some wall or some wall part is substantially transparent.

In an embodiment, said container comprises walls made from transparent glass and/or transparent polymer, preferably transparent polyimide (PI).

In an embodiment, said container is for the cultivation of a crop plant. In other embodiments, the container is for the cultivation of a houseplant, pot plant and/or indoor plant.

In an embodiment, the container is for soilless culture.

In an embodiment, the container if for hydroculture.

In an embodiment, the container is for hydroponic culture.

In an embodiment, the cultivation is cultivation in a glasshouse and/or indoor cultivation, preferably of a crop plant.

In an embodiment, the material has the property of reversibly changing colors when it gets in contact with water. The material thus also has the property of reversibly changing colors when water and or moisture that is in contact with the material is removed, for example by way of evaporation, diffusion, or absorption by components, plants and so forth in the container.

In an embodiment the material has a first color when it is in contact with liquid water and a second color when it is not in contact with liquid matter, wherein said first and second colors are different. In particular, the material reflects light having a different wavelength depending on whether or not it is in contact with water, for example liquid water, but in some embodiments also depending on whether or not it is in contact with moisture.

In a particular embodiment, the first color of the material is red and the second color is green.

In a preferred embodiment, the material is a hydrochromic material.

In an embodiment, the material comprises a cholesteric liquid-crystalline polymer layer.

In an embodiment, the material comprises a comprises a poly ampholyte layer. The poly ampholyte layer is preferably deposited on top of said cholesteric liquid-crystalline polymer layer.

In an embodiment, said cholesteric liquid-crystalline polymer layer is prepared by the polymerization of a an inert nematic mesogen in presence of a non-chiral nematic cross-linker and a chiral nematic cross-linker, and preferably a surfactant.

In an embodiment, the material that has the property of changing colors depending on exposure to water, such as liquid water and/or moisture, is used in the cultivation of a plant, preferably in association with a container in which said plant is cultivated.

The method also provides a method for cultivating a plant using the container and/or the arrangement comprising the container and the material.

In an embodiment, the method comprises providing a light sensor outside said container, said sensor being provided to capture light reflected by said material. Preferably, the sensor captures said light across said transparent wall part. In a further embodiment, the method comprises providing a light source for illuminating the material, preferably through said transparent wall part.

In an embodiment, the method comprises providing a data processing entity configured to determine, from a signal produced by said light sensor, the water status in said container and/or water requirements of a plant cultivated in said container. Preferably, based on said water status, the system is configured to produce an alarm and/or a warning in case said water status in below and/or beyond and optimal range, in particular a humidity range. For example, said alarm and/or warning is produced if there is lack of water in the container, if the plant is in water shortage, if the water status in the container is too dry, and the like.

In an embodiment, the system is configured to control an inlet of water into said container, or a sprinkler installation, wherein said system is further configured to trigger watering of the plant in said container depending on the water status and/or humidity in said container.

In an embodiment, the system is configured to directly act upon a watering system and induce the watering of the plant. For example, the data processing entity may be configured to command a sprinkler installation in an automated manner, so as to stop, start, or prevent watering via the sprinkler installation in dependence of the water status as determined by the system.

While certain of the preferred embodiments of the present invention have been described and specifically exemplified above, it is not intended that the invention be limited to such embodiments. Various modifications may be made thereto without departing from the scope and spirit of the present invention, as set forth in the following claims. Herein below, examples of the invention are disclosed. These examples are for illustration only and are not intended to limit the scope of the present invention.

### Examples

Examples of materials that have the property of changing their color in dependence of exposure to water or moisture are presented below based on cholesteric liquid-crystalline (CLC) or chiral nematic polymers. These are one-dimensional photonic materials which exhibit selective reflection of only one-handed circularly polarized light due to the presence of helical molecular organization with the wavelength of light reflected being directly proportional to length of the helical pitch. Responsive to the external stimuli, the pitch size changes giving rise to a visible appearance in color. We have applied one type of such materials to fabricate hydrochromic (HC) coatings, whose pitch size increases when in contact with water/moisture, leading to a red-shift of its characteristic reflection peak position.

### Example 1

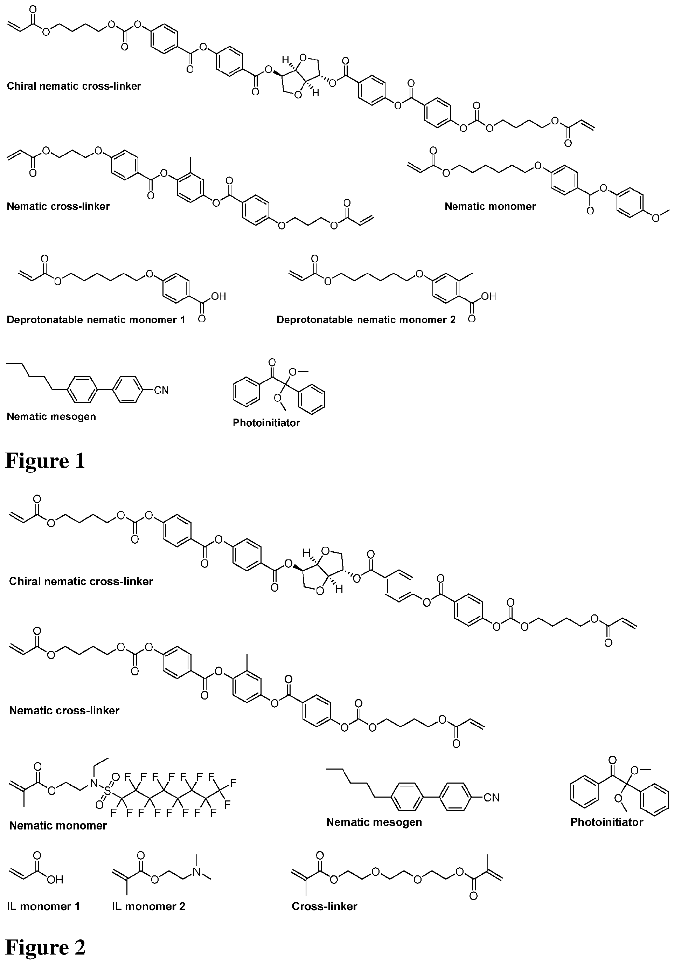

The materials shown in Figure 1 were mixed together according to the recipe listed in Table 1. The mixture contains (chiral) nematic cross-linkers and monomers for fulfilling the optical responsiveness. Some of them can be deprotonated for post-functionalization to increase the hydrophilicity. The inert nematic mesogen was used for the directionality and mesoporosity (subject to washing) of the obtained polymer films.

**Table 1: Recipe for the CLC polymer in Example 1 (Figure 1)**

| **Ingredient** | **Mol%** |
|---|---|
| **Chiral nematic cross-linker** | 1.7 |
| **Nematic cross-linker** | 10.6 |
| **Nematic monomer** | 20.0 |
| **Deprotonatable nematic monomer 1** | 21.4 |
| **Deprotonatable nematic monomer 2** | 20.4 |
| **Nematic mesogen** | 25.1 |
| **Photoinitiator** | 0.8 |

The hydrochromic CLC polymer film was prepared according to the following process:
A carefully cleaned glass plate (3×3 cm²) was surface-functionalized with 3-(trimethoxysilyl)propyl methacrylate, after which a 40 µL of solution containing the mixture in Table 1 and tetrahydrofuran as the solvent at a weight ratio of 1:2 was cast on top with the solvent was removed subsequently by heating at 75°C on a hotplate. Another piece of glass which was functionalized with 1H,1H,2H,2H-perfluorodecyltriethoxysilane solution was placed on top and sheared during cooling to create directionality. The whole assembly was then exposed to UV light (48 mW cm⁻² intensity in the range 320-390 nm) for 5 min and the top glass plate was removed to obtain the polymer coating. The polymer was first washed in THF and then treated with 1 M KOH to obtain the responsive potassium salt CLC polymer coating.

### Results

The films prepared according the above-mentioned procedure showed purple blue to green HC behaviors. However, due to the benzoate sites present the film, potential metal ions in the nutrient solutions may bind and lock the pitches, causing loss in the HC responsivity.

### Example 2

Another recipe was followed using ionic liquid monomer for realizing HC responsivity without introducing external ions. The film consists of an interpenetrating network between the poly(ampholyte) generated from the ionic liquid monomer and a CLC polymer that reflects light. The chemicals are shown in Figure 2 and listed in Table 2. As both cation and anion are incorporated in the ionic network, this coating possesses a high stability of its water responsiveness after prolonged and/or repeated exposure to water, even if the water contains dissolved ions. This turned out to be the case when testing the fabricated films in the physiological solution as mentioned in section 1) of Example 1.

**Table 2: Recipe for the HC polymers in Example 2.**

| **Ingredient** | **Mol%** |
|---|---|
| **Chiral nematic cross-linker** | 1.5 |
| **Nematic cross-linker** | 22.9 |
| **Nematic monomer** | 0.6 |
| **Nematic mesogen** | 73.6 |
| **Photoinitiator** | 1.4 |
| **Total, CLC polymer** | **100** |
| **IL monomer 1** | 49.6 |
| **IL monomer 2** | 49.6 |
| **Cross-linker** | 0.4 |
| **Photoinitiator** | 0.4 |
| **Total, poly(ampholyte)** | **100** |

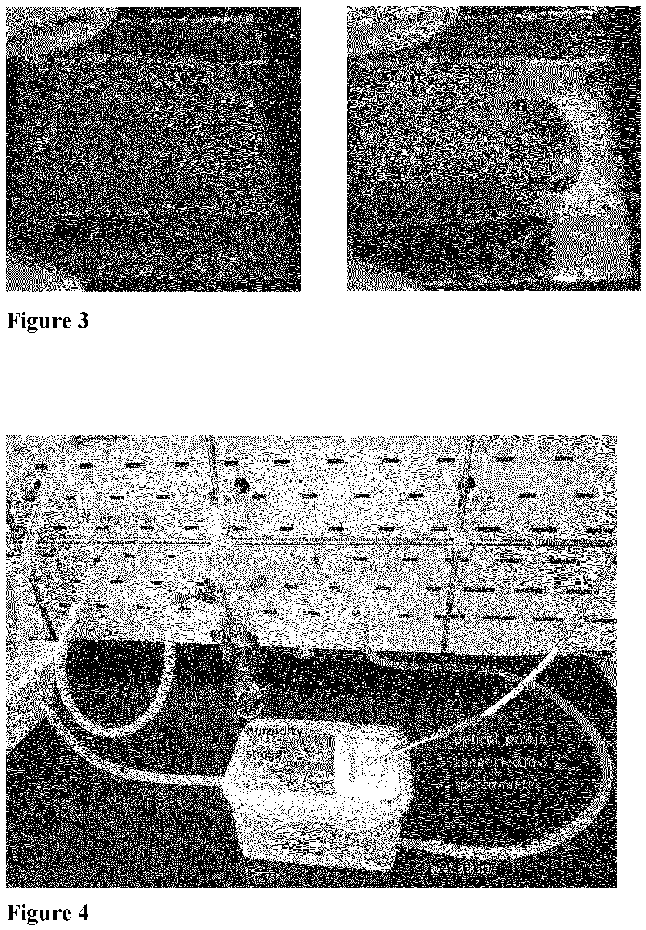

Like in Example 1, a CLC polymer was first prepared. The nematic chemicals and the photoinitiator shown in Figure 2 were mixed together according to the ratio listed in Table 2. The resulting paste was doctor-bladed on the glass substrate functionalized with 3-(trimethoxysilyl)propyl methacrylate and immediately photopolymerized under UV light (48 mW cm⁻² intensity in the range 320-390 nm) for 5 min. The nematic mesogen was washed off by immersion in tetrahydrofuran to result in the CLC polymer. It was then heated to 65°C on a hotplate and a mixture containing the ionic liquid monomers, the cross-linker, and the photoinitiator as a ratio listed in Table 2 was then cast on top and covered with another glass functionalized with 1H,1H,2H,2H-perfluorodecyltriethoxysilane After 30min, the top glass was removed and the excess of the ionic liquid monomers was cleaned off. The wet film was photopolymerized under UV light (48 mW cm⁻² intensity in the range 320-390 nm) for 5 min and then thoroughly rinsed with DI water. The dry film looked bright green which turned red when in contact with water (Figure 3).

### Film characterization

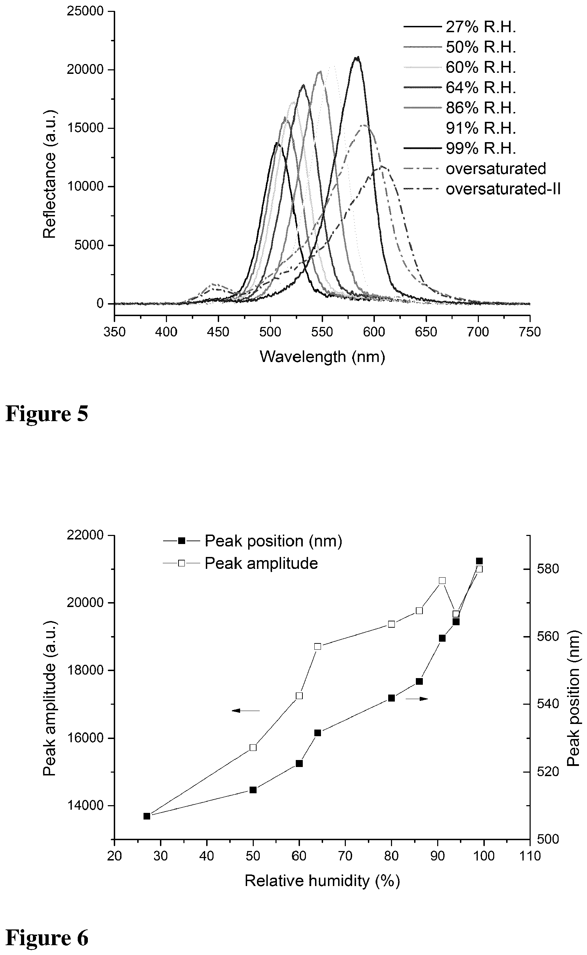

A home-made humidity chamber (Figure 4) was employed to monitor the change of the characteristic reflection peak position of the HC composite film as a function of different humidity levels. Figures 5 and 6 show the change of the reflectance peak position as a function of the humidity levels for the film shown in Figure 3. A monotonic increase of both the peak wavelength and the reflectance intensity was observed.

### Stability

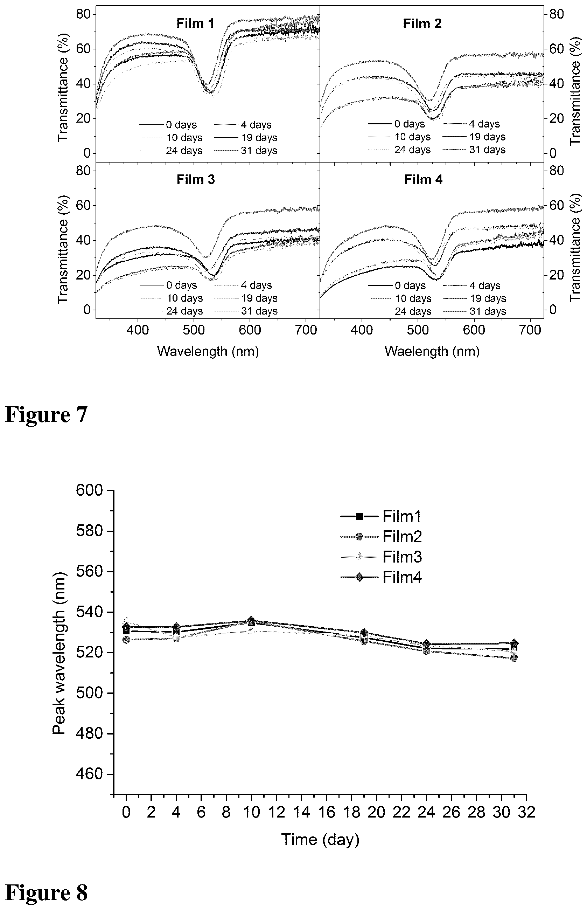

The stability of the films in the nutrient solution as mentioned in Example 1 was tested. The films were taken out of the solution at intervals and the transmittance spectra were recorded after rinsing and drying. The results are plotted in Figure 7 and the trend is shown in Figure 8. Judging from the trend of the peak position evolution, a considerable stability of the HC composite film could be concluded.

### Example 3: Hydrochromic coatings on transparent plastic substrates

Fabrication of the HC composite films on plastic substrate were attempted. Polyimide (PI) films (31 µm, from Nagase & Co., Ltd.) were cut into 2×4 cm² pieces and functionalized with the presence of aminopropylmethacrylamide hydrochloride and tributylamine for 24 hr. Reflective green-to-red HC films can be successfully fabricated using the method described in Example 2 except with the plastic PI substrate.

### Example 4: Plant containers comprising hydrochromic material coatings

In a first experiment, the flexible PI substrate produced in Example 3 is used to prepare a plastic container. In particular, the square PI substrate comprising the hydrochromic coating is glued to other PI squares of identical thickness but lacking the hydrochromic coating so as to form a cube lacking the top wall.

The container is filled with clay pebbles, lettuce seedlings are inserted and water supplied with fertilizer is added to the container. On the side wall comprising the hydrochromic coating, the color indicated the level of the added water in the container, with red color indicating the presence of water and green color indicating the absence of water in the container. The limit between the red and green colors could be used to directly read the water level in the container.

In a second embodiment, a pre-existing rectangular glass container was used. The square PI substrate comprising the hydrochromic coating prepared as described in Example 3 was inserted along a transparent sidewall of the container, such that the hydrochromic coating was visible from the outside through the transparent walls of the container. The container was filled with clay pebbles were added as described above, such that the pebbles kept the PI substrate towards the transparent container wall. As with the previous experiment, lettuce seedlings are inserted and water supplied with fertilizer is added to the container. The water status in the container could be read from the color on the PI substrate visible through the transparent container wall. Once all water was used up, red color was absent from the substrate and the substrate was mostly green.

## Claims

1. A container for the cultivation of a plant, the container comprising at least one transparent wall part and a material that has the property of exhibiting a particular color depending on the humidity to which said material is exposed, wherein said material is associated with said container so as to be visible through said transparent wall part.

2. The container of claim 1, wherein said material is provided in the form of a coating on the inner side of said transparent wall part.

3. The container of claim 1, wherein said material is provided on a substrate provided inside the container said substrate being visible through said transparent wall part.

4. The container of claim 1, wherein said material has the property of reversibly changing colors when it gets in contact with water.

5. The container of claim 1 or claim 2, wherein said material is a hydrochromic material.

6. The container of any one of the preceding claims, wherein said transparent wall part is preferably a transparent side wall part, preferably a transparent side wall.

7. The container of any one of the preceding claims, which comprises walls made from transparent glass and/or transparent polymer, preferably transparent polyimide (PI).

8. The container of any one of the preceding claims, which is substantially made from a transparent material.

9. The container of any one of the preceding claims, which is for the cultivation of a crop plant.

10. The container of any one of the preceding claims, which is for soilless culture and/or hydroculture, preferably hydroponic culture.

11. The container of any one of the preceding claims, wherein said cultivation is indoor cultivation and/or cultivation in a glasshouse.

12. The container of any one of the preceding claims, wherein said material preferably comprises a cholesteric liquid-crystalline polymer layer.

13. The container of any one of the preceding claims, wherein said material comprises a poly ampholyte layer, preferably deposited on top of said cholesteric liquid-crystalline polymer layer.

14. The container of any one of claims 11 and 12, wherein said cholesteric liquid-crystalline polymer layer is prepared by the polymerization of a an inert nematic mesogen in presence of a non-chiral nematic cross-linker and a chiral nematic cross-linker, and preferably a surfactant.

15. Use of an arrangement comprising a substrate and a material having the property of exhibiting a particular color depending on the humidity to which said material is exposed for indicating water status in the cultivation of a plant, preferably in soilless culture and/or hydroculture.

16. A method for cultivating a plant, the method comprising:
- providing the container according to any one of claims 1-13;
- cultivating a plant in said container;
- monitoring humidity and/or water status in said container by monitoring the color and/or wavelength of the light reflected by said material.

17. The method of claim 16, wherein monitoring humidity comprises providing a light sensor outside said container, said sensor being provided to capture light reflected by said hydrochromic coating.

18. The method of claim 17, comprising providing a data processing entity configured to determine, from a signal produced by said light sensor, the water status in said container and/or water requirements of a plant cultivated in said container.

19. A system suitable for monitoring water status in the cultivation of plants, the system comprising:
- the container of any one of claims 1-14;
- a sensor provided to capture light reflected by the hydrochromic coating associated with said container;
- a data processing entity configured to determine, from a signal produced by said light sensor, the humidity and/or water status in said container and/or water requirements of a plant cultivated in said container.

20. The system of claim 19, which is configured to control an inlet of water into said container, or a sprinkler installation, wherein said system is further configured to trigger watering of the plant in said container depending on the water status and/or humidity in said container.
